# EUROPEAN PATENT SPECIFICATION

(11) **EP 0 890 648 B1**
(45) Date of publication and mention of the grant of the patent: **17.10.2001**
(21) Application number: 97304977.8
(22) Date of filing: 08.07.1997
(51) Int. Cl.: C12P 1/04, C12P 19/04, A61K 35/74

(54) **Non-toxic lipopolysaccharide and its preparation**
Nicht-toxisches Lipopolysaccharid und seine Herstellung
Lipopolysaccharide non toxique et sa préparation

(43) Date of publication of application: 13.01.1999
(73) Proprietor: Council of Scientific & Industrial Research, New Delhi 110001 (IN)
(72) Inventor: Bhadra, Ranjan, Calcutta-700 032 (IN); Nayak, Abhijit, Calcutta-700 032 (IN); Bandyopadhyay, Pataki C., Calcutta-700 032 (IN); Basu, Sumanta, Calcutta-700 032 (IN)
(74) Representative: Towler, Philip Dean

(56) References cited:
- WO-A-94/25476
- SUMANTA BASU ET AL.: "Lipopolysaccharides of the acidophilic heterotrophic bacteria Acidiphilium cryptum and Acidiphilium symbioticum." FEMS MICROBIOL. LETT., vol. 118, no. 1-2, 1994, pages 65-69, XP002049576
- DATABASE WPI Section Ch, Week 9648 Derwent Publications Ltd., London, GB; Class B04, AN 96-482261 XP002049577 & JP 08 245 702 A (MIZUNO D) , 24 September 1996

## Description

This invention relates to a process for the preparation of a new potent non-toxic lipopolysaccharide (LPS) useful for preventing endotoxemia or sepsis.

The bilayered and asymmetrically organised membrane, the so called outer membrane, of Gram negative bacteria such as Escherichia coli, Salmonella enteriditis contains certain proteins and a class macroamphiphiles. The LPS are dominating constituents. The viability of these bacteria is solely dependent on the array of LPS on the outer membrane of the cell. One bacterial cell contains approximately 3.6x10⁶ LPS molecules occupying an area of 4.9 micro square meters. It appears that for this its interaction is easy with the target.

Upon infection with a Gram negative pathogen, the LPS released in the circulation mediates various physiological and pathophysiological interactions with a host such as a human, leading to the risk to its survival. The pathophysiological disorders caused by LPS of Gram negative bacterial infections in human include fever (about 38.6°C), tachycardia (>90 beats/ min.), lachypnea (respiratory rates >20 breath/ min.), acidosis (pH 7.3) and arterial hypoxemia (ppo = 75 mm of Hg). In the United States alone every year 500,000 people suffer sepsis and 175,000 of them die. In India the problem is more serious because of the higher population and there is a higher rate of septicemia with greater impact on the mortality rate. Usually patients in hospital or intensive care units having an incidence of septic shock die within 72 hours of diagnosis.

Amongst the mediators that are produced by LPS action on reticuloendothelial cells are tumour necrosis factor (TNF) and interleukin-1 (IL-1) in addition to interleukin 6 and 8. However TNF alpha and IL-1 are critical factors for septicemia.

The market for successful antisepsis drugs could be $500 million a year according to Oppenheimer & Co. Inc. of New York City (4). However, the development of antisepsis drugs is not easy.

Starting in mid 1980s, Centocor, Xoma and Chiron of Emeryville, California developed a monoclonal antibody to lipid-A, the toxic component of endotoxin that plays key role in generating sepsis. By binding to receptors on the monocytes and macrophages, the lipid-A of endotoxin triggers the release of powerful mediators known as cytokines. The idea of using antibodies to collect excess of lipid-A before it can cause sepsis was exploited by Xoma and Centocor in their products in clinical trials, but without success. However RihiImmune Chemical of Hamilton, Montana developed a benign form of lipid-A which tolerates the host and the immune system no longer becomes overstimulated when there is infection with Gram negative and positive bacteria. Another approach for anti-endotoxin activity is use of a bactericidal/permeability increasing protein (BPI), a self protein present in white blood cells which binds to endotoxin and thereby mimics body's own action. This was developed by Incyte Pharmaceuticals of Palo Alto, California, and has shown good results in mice. Due to shift in the pathogens causing sepsis such as Gram positive bacteria and fungi, focus is now directed on immune mediators such as TNF and IL-1.

Synergen's Antril (receptor antagonist of IL-1) is a protein produced by monocytes and helps in counteracting the effect of IL-1 by binding to its receptor on neutrophils. This worked well in patients who were already ill. Several companies such as Chiron, England, Hoffmann La Roche Ltd, Switzerland, had encouraging results using TNF-binding antibodies and are now testing in humans.

However anti TNF receptors approach was not successful as it has been reported to increase mortality in sepsis patients. The anticytokine approach still has plenty of defenders but others complain that it triggers only one chemical reaction at a time. Nevertheless, controlling one factor increases the effect of others. Thus if TNF is blocked there is enough redundancy in the immune system that IL-1 level probably goes up.

Successful therapy against sepsis will probably require a cocktail of agents that works against both TNF and IL-1. EntreMed Inc. in Rockville, Maryland has moved in this direction by using an "antisepsis vaccine" to get patients to mount their own immune response. The vaccine consists of lipid-A sheathed in vesicles made of lipids and cholesterol is in development. Therefore a possible approach in the direction of an "antisepsis vaccine" is attempted with a naturally occurring non-toxic LPS which is cross-reactive immunologically with E. coli LPS but would show no lethality at a very high dose. On the contrary it prevents lethal shock induced by Gram negative toxic LPS or endotoxin.

FEMS Microbiology Letters, 118 (1994) 65-70 describes the use of two strains of Acidiphilium to produce lipopolysaccharides. These strains and lipopolysaccharides are different from those of this invention. JP 8245702 describes a high molecular weight lipopolysaccharide obtained from Pantoea strains and its use to enhance vaccine effects. WO 94/25476 describes proteins which act on toxic lipopolysaccharides produced by bacteria. In the state of art there are various standard processes for producing LPS.

### A. 1. Extraction with trichloroacetic acid

Extraction of the cell with trichloroacetic acid (TCA) represents historically the first method for isolation of endotoxin. This is a highly immunogenic LPS protein-phospholipids complex.

Cells suspended in five times their weight of ice cold water are treated with an equal volume of 0.5 N TCA at 4°C for 3 hours. The suspension is centrifuged, the supernatants neutralised with dilute sodium hydroxide, and the complex precipitated with two volumes of water, dialysed, centrifuged to remove the cell debris and lyophilised.

### 2. Extraction with ethylene glycol

Diethylene glycol selectively extracts free polysaccharide and/or the whole O-antigen complex from cells. The extraction conditions are very mild and the risk of degradation or modification of the extracted material is low. Acetone dried cells are extracted with 10 parts of freshly distilled diethylene glycol by shaking for several hours each day at room temperature. The extract is filtered, dialysed and concentrated. The O-antigen complex is finally precipitated by addition of acetone at -10°C. Further purification is done by fractionation with a 1% aqueous solution of saturated ammonium sulphate. The procedure is lengthy and the yield is low.

### 3. Extraction with hot phenol

Although the extraction methods described above are useful, the isolation procedure worked out by Westphal and Jann in 1965 is mostly used. The material extracted is slightly contaminated with protein. Method works well for extraction of S and R type of LPS although it is often not complete for R mutants.

### 4. Extraction with dimethyl sulphoxide (DMSO)

At higher temperature (>60°C) this extracts the LPS with protein. From this extract LPS free of protein can be obtained by O-acetylation and subsequently purified with chloroform. Finally, the product is deacylated with DMSO (2% in acetone at room temperature). It is possible that part of ester-linked fatty acids is removed from lipid-A during deacylation.

### 5. Extraction with phenol/chloroform/petroleum ether (PCP)

The phenol/water method often does not extract quantitatively the more hydrophobic endotoxin present in R mutants. In that case, dried bacteria are homogenised in a mixture of liquid phenol-chloroform-petroleum ether (2:5:8 v/v/v), then centrifuged and the supernatant is collected. Chloroform and petroleum ether is removed by evaporation from supernatant. Water is added dropwise to precipitate LPS in remaining phenol. The precipitate is centrifuged followed by washing with petroleum ether and acetone with further purification by ultracentrifugation at 105,000 rpm for 4 hours.

### B. Electrodialysis

Isolated endotoxin from both S and R forms are usually associated with a number of mono and divalent cations such as Na+, K+, Mg++, Ca++ and some polyamines, e.g. spermidine and spermine. These cations have a strong influence on the solubility and aggregation of LPS. Electrodialysis of LPS enables its conversion to salt form by neutralisation with bases. The triethylamine salt form of LPS is highly water soluble. This salt is not sedimented in the ultracentrifugation and shows modified biological activities. Unneutralised electrodialysed LPS preparations are not stable for a long time and deteriorate on storing because of autolysis.

The lethal dose of toxic LPS of E. coli is 80 microgram/mouse and the lethal dose of non-toxic LPS of present invention is 3 mg/mouse in non galactosamine sensitised mice. Other non-toxin LPS such as those of R.sphearoides and R.capsulatus are reported to be pyrogenic and lethal at dose not higher than the newly found one.

The main objective of the present invention is to provide a process for the preparation of a new lipopolysaccharide from a new strain belonging to the genus Acidophilium isolated from the soil in the areas of copper mines.

Another objective of the invention is to provide a process for the preparation of a new lipopolysaccharide as an intervening agent for toxic LPS action culminating in endotoxemia, but itself being non-toxic.

Accordingly, the present invention provides a process for the preparation of a new non-toxic lipopolysaccharide, useful in the treatment of endotoxin shock, comprising:
1) culturing bacteria of the genus Acidiphilium having accession No. MTCC No. 1979;
2) separating the cultured bacteria followed by repeated washing;
3) extracting the washed bacteria with a lipophilic solvent;
4) cooling the resultant extract and centrifuging it repeatedly;
5) dialysing the supernatant resulting from the step 4;
6) lyophilising the dialysed material obtained from step 5;
7) treating the lyophilised material with a polar solvent to precipitate the lipopolysaccharide; and
8) collecting the lipopolysaccharide.

The lipopolysaccharide prepared by the process of the invention is a bioactive substance and useful for preventing pathophysiological interaction with endotoxins of Gram negative bacteria including human pathogens. It is 150-200 times less toxic than the known non-toxic LPS and in particular is 2 million times less toxic than E.coli LPS and 150-200 times less toxic than R.sphaeorides. This non-toxic LPS is useful as preventive agent against endotoxemia.

The invention also includes the new non-toxic lipopolysaccharide obtainable by the process of the invention, and pharmaceutical compositions containing the lipopolysacchride together with one or more pharmaceutical carriers or excipients.

The new strain MTCC No. 1979 has been deposited with the National Culture Centre IMTFC, Chandigargh, India. The same strain has also been deposited with the American Type Culture Collection on 30 April 1997 as Acidiphilium GS18h and has been assigned the deposit number ATCC 55963.

The bacterial culture may be grown in a conventional nutrient medium (e.g. a synthetic media enriched with glucose and yeast extract) and incubated under standard conditions, e.g. at a temperature in the range of 30-39°C for a period in the range of 72-120 hours.

The nutrient medium may contain other hexoses such as galactose, and also sulphates such as ammonium sulphate and minerals such as magnesium or dipotassium hydrogen phosphate and potassium chloride.

Following the growth of cells they are harvested for example by centrifugation at 4000 to 10,000 rpm for 30 to 100 minutes, followed by washing for example with the same medium employed for growth but not containing glucose and yeast extract. Other methods of separation may also be used, such as sharple centrifugation, membrane filtration or gravitational sedimentation.

Bacterial cells are then treated with lipophilic solvent such as an aromatic phenol (e.g. phenol itself) or a C₁₋₄ alkanol (e.g. butanol), and water. The solvent is preferably used hot, e.g. at 70-90°C. The mixture is then stirred vigorously e.g. for 60-120 minutes. The mixture is then cooled to 2-12°C in ice and then centrifuged at 4000 to 10,000 rpm for 15 to 30 minutes.

The above procedure is for example repeated three times. The total supernatant is exhaustively dialysed, e.g. for 2-5 days. The material is then lyophilised and subjected to ultracentrifugation at 105,000 to 170,000 rpm for 4-6 hours at a temperature in the range of 2-4°C in the form of a 1.5-7.5% solution. The water used in the entire process should be fully pyrogen free and care should be taken to avoid any contamination from the environment. The centrifugation is repeated in order to obtain nearly pure LPS. This is then treated with polar solvent (e.g. a C₁₋₄ alkanol such as ethanol) to give a precipitate which can be collected for example by centrifugation at 8500 to 14000 rpm for 20-45 minutes and lyophilised for storage in a refrigerator.

The non-toxic LPS (NTLPS) which is prepared in this invention is a unique type of LPS from an Acidiphilium bacteria and different from known strains of Acidiphilium as source of NTLPS.

Usually the basic structure of the LPS lipid-A has 4 to 8 acyl chains of varying length. In a comparative study of the structure forms function relation of LPS, various modified forms E.coli LPS have been illustrated and their bioactivity showed some striking features. More beta-hydroxylation and a lower number of fatty acyl chain results in drastically reduced bioactivity, by a factor of 10⁷. The non-toxic LPS prepared in this invention has a higher proportion of beta-hydroxylated fatty acid acyl chains, namely myristic acid chains, which possibly accounts for its non-toxicity, though the NTLPS is cross-reactive with E.coli LPS immunologically.

The composition of this non-toxic LPS (ie. GS18h LPS from MTCC 1979 strain) is as follows:

| A. | SUGAR | % OF TOTAL |
|---|---|---|
| | Galactose | 77.3 |
| | Glucose | 9.06 |
| | Mannose | 3.4 |
| | L-D Heptose | 1.2 |

| B. | FATTY ACID | % OF TOTAL |
|---|---|---|
| | R-OH-C_{14:0} | 58.3 |
| | C_{16:0} | 11.95 |
| | C_{12:0} | 3.75 |
| | C_{16:1} | 14.63 |

The non-toxic LPS of the present invention is composed of electrophoretically low molecular weight components as compared to E.coli LPS electrophoretically. The high degree of its immunological cross-reactivity with E.coli LPS suggests it is very closely related structurally with E.coli LPS.

The invention is described in detail in the examples below.

### Example 1

A strain of MTCC 1979 was cultured, harvested and weighing 20g (wet weight) 4g (dry weight) under following conditions:

| | |
|---|---|
| Volume of culture (2 days) | 5 litres |
| Weight of bacterial cell | 4g (dry wt.) |
| Volume of pyrogen free water | 70 ml |
| Volume of distilled phenol | 70 ml |
| Temperature | 68°C |
| Duration of stirring | 30 minutes |
| Speed of centrifugation | 105,000 rpm |
| Duration of centrifugation | 4 hours |
| Crude LPS obtained | 100 mg |
| Purified LPS obtained | 75 mg |

### Example 2

| | |
|---|---|
| Volume of culture (2 days) | 5 litres |
| Weight of bacteria cell | 6g (dry wt.) |
| (with higher concentration of glucose) | (2g/litre) |
| Volume of pyrogen free water | 70 ml |
| Volume of distilled phenol | 70 ml |
| Temperature | 68°C |
| Duration of stirring | 30 minutes |
| Speed of centrifugation | 105,000 rpm |
| Crude LPS obtained | 130 mg |
| Purified LPS obtained | 105 mg |

### Example 3

| | |
|---|---|
| Volume of culture (2 days) | 2.5 litres |
| Weight of bacteria cell | 2g |
| Volume of pyrogen free water | 35 ml |
| Volume of distilled phenol | 35 ml |
| Temperature of extraction | 75°C |
| Duration of centrifugation | 30 minutes |
| Speed of centrifugation | 105,000 rpm |
| Duration of centrifugation | 4 hours |
| Crude LPS obtained | 52 mg |
| Purified LPS obtained | 38 mg |

Experimental evidence of non-toxic nature of the lipopolysaccharide prepared by the process of the present invention.

Two groups of mice, each consisting of 5 animals, were taken for survival test upon administration of the lipopolysaccharide prepared in this invention and that of E.coli.

In one group only normal saline was injected and in other group LPS was injected. No death was observed in 5 days. On the fifth day the toxic LPS was injected into both the groups. The saline treated group died within 72 hours but non-toxic lipopolysaccharide primed mice survived totally without showing any mortality. All injections were given after mild anaesthesia to mice.

## Claims

1. A process for the preparation of new non-toxic lipopolysaccharide (LPS) useful in the treatment of endotoxemia which comprises:
1) culturing bacteria of the genus Acidiphilium having accession No. MTCC No. 1979 (ATCC 55963);
2) separating the cultured bacteria followed by repeated washing;
3) extracting the washed bacteria with a lipophilic solvent;
4) cooling the resultant extract and centrifuging it repeatedly;
5) dialysing the supernatant resulting from the step 4;
6) lyophilising the dialysed material obtained from step 5;
7) treating the lyophilised material with a polar solvent to precipitate the lipopolysaccharide; and
8) collecting the lipopolysaccharide.

2. A process as claimed in claim 1 wherein nutrient medium contains hexoses such as glucose or galactose as a carbon source, a nitrogen source such as yeast extract, ammonium sulphate, and a mineral such as magnesium or dipotassium hydrogen phosphate or potassium chloride.

3. A process as claimed in claim 1 or claim 2 wherein the separation of the bacteria is effected by centrifuging at 4000-10,000 rpm or by sharple centrifugation, membrane filtration or slow gravitational sedimentation.

4. A process as claimed in any preceding claim wherein the lipophilic solvent used includes phenol or butanol and water.

5. A process as claimed in any preceding claim in which the extraction (3) is carried out at 70-90°C.

6. A process as claimed in any preceding claim wherein the polar solvent used for precipitation is a C₁₋₄ alkanol.

7. A non-toxic lipopolysaccharide obtainable by a process as claimed in any preceding claim.

8. A non-toxic lipopolysaccharide having the following composition:
| A. | SUGAR | % OF TOTAL |
|---|---|---|
| | Galactose | 77.3 |
| | Glucose | 9.06 |
| | Mannose | 3.4 |
| | L-D Heptose | 1.2 |
| B. | FATTY ACID | % OF TOTAL |
|---|---|---|
| | R-OH-C_{14:0} | 58.3 |
| | C_{16:0} | 11.95 |
| | C_{12:0} | 3.75 |
| | C_{16:1} | 14.63 |

9. A pharmaceutical composition containing a lipopolysaccharide as claimed in claim 7 together with one or more pharmaceutical carriers or excipients.

10. Acidiphilium GS18h (ATCC 55963).

## Patentansprüche

1. Verfahren zur Herstellung eines neuen, nichttoxischen Lipopolysaccharids (LPS), das sich zur Behandlung von Endotoxämie eignet, **dadurch gekennzeichnet, daß** man:
1) Bakterien der Gattung Acidiphilium mit der Zugangs-Nr. MTCC Nr. 1979 (ATCC 55963) kultiviert;
2) die kultivierten Bakterien trennt und anschließend mehrfach wäscht;
3) die gewaschenen Bakterien mit einem lipophilen Lösungsmittel extrahiert;
4) den so erhaltenen Extrakt abkühlt und mehrfach zentrifugiert;
5) den in Schritt 4 erhaltenen Überstand dialysiert;
6) das in Schritt 5 erhaltene dialysierte Material lyophilisiert;
7) das lyophilisierte Material mit einem polaren Lösungsmittel behandelt, um das Lipopolysaccharid auszufällen; und
8) das Lipopolysaccharid sammelt.

2. Verfahren nach Anspruch 1, bei dem das Nährstoffmedium Hexosen wie z.B. Glukose oder Galaktose als Kohlenstoffquelle, eine Stickstoffquelle wie z.B. Hefeextrakt, Ammoniumsulfat und einen Mineralstoff wie z.B. Magnesium- oder Dikaliumhydrogenphosphat oder Kaliumchlorid enthält.

3. Verfahren nach Anspruch 1 oder 2, bei dem die Trennung der Bakterien durch Zentrifugieren bei 4000 - 10 000 U/min oder durch Sharpel-Zentrifugation, Membranfiltration oder langsame Schwerkraftssedimentation erfolgt.

4. Verfahren nach einem der vorhergehenden Ansprüche, bei dem das verwendete lipophile Lösungsmittel Phenol bzw. Butanol und Wasser enthält.

5. Verfahren nach einem der vorhergehenden Ansprüche, bei dem die Extraktion (3) bei 70-90°C durchgeführt wird.

6. Verfahren nach einem der vorhergehenden Ansprüche, bei dem es sich bei dem für die Ausfällung verwendeten polaren Lösungsmittel um ein C₁₋₄-Alkanol handelt.

7. Nicht-toxisches Lipopolysaccharid, erhältlich durch ein Verfahren nach einem der vorhergehenden Ansprüche.

8. Nicht-toxisches Lipopolysaccharid der folgenden Zusammensetzung:
| A. | ZUCKER | % DER GESAMTMENGE |
|---|---|---|
| | Galaktose | 77,3 |
| | Glukose | 9,06 |
| | Mannose | 3,4 |
| | L-D Heptose | 1,2 |
| B. | FETTSÄURE | % DER GESAMTMENGE |
|---|---|---|
| | R-OH- C_{14:0} | 58,3 |
| | C_{16:0} | 11,95 |
| | C_{12:0} | 3,75 |
| | C_{16:1} | 14,63 |

9. Pharmazeutische Zusammensetzung, enthaltend ein Lipopolysaccharid nach Anspruch 7 zusammen mit einem oder mehreren pharmazeutischen Träger- bzw. Füllstoffen.

10. Acidiphilium GS18h (ATCC 55963).

## Revendications

1. Procédé de préparation d'un nouveau lipopolysaccharide (LPS) non toxique utile dans le traitement de l'endotoxémie, qui comprend les étapes consistant à :
1) cultiver des bactéries du genre Acidiphilium ayant un N° de référence MTCC 1979 (ATCC 55963) ;
2) séparer les bactéries cultivées, puis les laver de façon répétée ;
3) extraire les bactéries lavées avec un solvant lipophile ;
4) refroidir l'extrait résultant et le centrifuger de façon répétée ;
5) dialyser le surnageant résultant de l'étape 4 ;
6) lyophiliser la matière dialysée obtenue dans l'étape 5 ;
7) traiter la matière lyophilisée avec un solvant polaire pour précipiter le lipopolysaccharide ; et
8) recueillir le lipopolysaccharide.

2. Procédé selon la revendication 1, dans lequel le milieu nutritif contient des hexoses, tels que le glucose ou le galactose, en tant que source de carbone, une source d'azote telle qu'un extrait de levure, le sulfate d'ammonium, et un composé minéral tel que l'hydrogénophosphate de magnésium ou dipotassique ou le chlorure de potassium.

3. Procédé selon la revendication 1 ou la revendication 2, dans lequel la séparation des bactéries a lieu par centrifugation à 4 000 - 10 000 tours par minute ou par centrifugation nette, filtration sur membrane ou sédimentation lente par décantation.

4. Procédé selon l'une quelconque des revendications précédentes, dans lequel le solvant lipophile utilisé comprend du phénol ou du butanol et de l'eau.

5. Procédé selon l'une quelconque des revendications précédentes, dans lequel l'extraction (3) est réalisée à une température de 70 à 90°C.

6. Procédé selon l'une quelconque des revendications précédentes, dans lequel le solvant polaire utilisé pour la précipitation est un alcanol en C₁-C₄.

7. Lipopolysaccharide non toxique pouvant être obtenu par un procédé selon l'une quelconque des revendications précédentes.

8. Lipopolysaccharide non toxique ayant la composition suivante :
| A. | SUCRE | % DU TOTAL |
|---|---|---|
| | Galactose | 77,3 |
| | Glucose | 9,06 |
| | Mannose | 3,4 |
| | L-H Heptose | 1,2 |
| B. | ACIDE GRAS | % DU TOTAL |
|---|---|---|
| | R-OH en C_{14:0} | 58,3 |
| | C_{16:0} | 11,95 |
| | C_{12:0} | 3,75 |
| | C_{16:1} | 14,63 |

9. Composition pharmaceutique contenant un lipopolysaccharide selon la revendication 7 avec un ou plusieurs véhicules ou excipients pharmaceutiques.

10. Acidiphilium GC18h (ATCC 55963).
